# EUROPÄISCHE PATENTSCHRIFT

(11) **EP 1 951 651 B1**
(45) Veröffentlichungstag und Bekanntmachung des Hinweises auf die Patenterteilung: **30.09.2009**
(21) Anmeldenummer: 06819417.4
(22) Anmeldetag: 13.11.2006
(51) Int. Cl.: C07C 57/02, C07C 57/13, D21H 17/16

(54) **ALKENYLBERNSTEINSÄUREANHYDRIDE AUS OLIGOMONEREN VON C 4- BIS C 8-OLEFINEN UND MALEINSÄUREANHYDRID, VERFAHREN ZU IHRER HERSTELLUNG UND IHRE VERWENDUNG**
ALKENYLSUCCINIC ANHYDRIDES FORMED FROM OLIGOMERS OF C4- TO C8-OLEFINS AND MALEIC ANHYDRIDE, PROCESSES FOR THEIR PREPARATION AND THEIR USE
ANHYDRIDE DE L ACIDE ACETYLSALICYLIQUE ISSU D OLIGOMONOMERES D OLEFINES EN C4 A C8 ET D ANHYDRIDE DE L ACIDE MALEIQUE, SON PROCEDE DE FABRICATION ET SON UTILISATION

(30) Priorität: 18.11.2005 DE 102005055541
(43) Veröffentlichungstag der Anmeldung: 06.08.2008
(73) Patentinhaber: BASF SE, 67056 Ludwigshafen (DE)
(72) Erfinder: LANGE, Arno, 67098 Bad Dürkheim (DE); CSIHONY, Szilard, 69469 Weinheim (DE); GASCHLER, Wolfgang, 67063 Ludwigshafen (DE); SCHMID, Markus, 67146 Deidesheim (DE)
(86) Internationale Anmeldenummer: PCT/EP2006/068375
(87) Internationale Veröffentlichungsnummer: WO 2007/057369

(56) Entgegenhaltungen:
- EP-A- 1 457 475
- EP-A1- 0 548 835
- DE-A1- 3 404 068
- DE-A1- 3 404 071

## Beschreibung

Die vorliegende Erfindung betrifft Alkenylbernsteinsäureanhydride aus Oligomoneren von C₄- bis C₈-Olefinden und Maleinsäureanhydrid, Verfahren zu ihrer Herstellung und ihre Verwendung als Leimungsmittel für Papier, Pappe und Karton.

Kohlenwasserstoffgemische, die kurzkettige Olefine, z. B. mit 2 bis 6 Kohlenstoffatomen enthalten, sind im großtechnischen Maßstab erhältlich. So fällt z. B. bei der Aufarbeitung von Erdöl durch Steamcracken oder Fluidized Catalyst Cracking (FCC) ein als C₄-Schnitt bezeichnetes Kohlenwasserstoffgemisch mit einem hohen Gesamtolefingehalt an, wobei es sich im Wesentlichen um Olefine mit 4 Kohlenstoffatomen handelt. Solche C₄-Schnitte, d. h. Gemische aus isomeren Butenen und Butanen, eignen sich, gegebenenfalls nach einer vorherigen Abtrennung des Isobutens und Hydrierung des darin enthaltenen Butadiens, sehr gut zur Herstellung von Oligomeren, insbesondere von Octenen und Dodecenen.

Die aus Olefingemischen mit überwiegend linearen Ausgangsolefinen erhältlichen Oligomerengemische, die im Wesentlichen linear sind, haben eine große Bedeutung erlangt. Sie eignen sich z. B. als Dieselkraftstoffkomponente sowie als Zwischenprodukte zur Herstellung funktionalisierter, überwiegend linearer Kohlenwasserstoffe. So erhält man durch Hydroformylierung und anschließende Hydrierung der Olefinoligomere die entsprechenden Alkohole, die unter anderem als Ausgangsstoffe für Detergenzien und als Weichmacher verwendet werden. Für viele Einsatzbereiche, z. B. als Weichmacheralkohole, spielt der Verzweigungsgrad der Olefine eine entscheidende Rolle. Der Verzweigungsgrad wird dabei beispielsweise durch den ISO-Index beschrieben, der die mittlere Zahl der Methylverzweigungen der jeweiligen Olefinfraktion angibt. So tragen z. B. bei einer C₈-Fraktion die n-Octene mit 0, Methylheptene mit 1 und Dimethylhexene mit 2 zum ISO-Index der Fraktion bei. Je niedriger der ISO-Index ist, um so größer ist die Linearität der Moleküle in der jeweiligen Fraktion.

Es ist bekannt, zur Herstellung wenig verzweigter, ebenfalls olefinisch ungesättigter Oligomere aus niederen Olefinen Katalysatoren einzusetzen, die als aktive Komponente Metalle und ganz überwiegend Nickel enthalten. Heterogene Katalysatoren weisen dabei gegenüber homogenen den Vorteil auf, dass eine Abtrennung des Katalysators vom Reaktoraustrag entfällt. So ist z. B. aus der DE-A-43 39 713 (= WO 95/14647) ein Verfahren zum Oligomerisieren von unverzweigten C₂- bis C₆-Olefinen an einem Festbettkatalysator bei erhöhtem Druck und erhöhter Temperatur bekannt, wobei der Katalysator als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumoxid enthält. Weitere Oligomerisierungskatalysatoren und -verfahren sind z. B. in der WO 99/25668, WO 00/59849, WO 00/53546, WO 01/72670 und EP-A 1 457 475 beschrieben.

Neben Wasser zählen sogenannte Ölkörper zu den wichtigsten Rohstoffen bei der Formulierung kosmetischer und pharmazeutischer Mittel. Die WO-A-2004/091555 beschreibt ein kosmetisches Mittel, das wenigstens ein verzweigtes α-Olefin oder ein Hydrierungsprodukt davon enthält. Dabei weist das α-Olefin wenigstens eine C₂- oder längerkettige Alkylverzweigung auf. Es wird durch Oligomerisierung bestimmter linearer oder verzweigter α-Olefine in Gegenwart eines sauren Katalysators hergestellt. Nachteilig an diesen Produkten ist ihr hoher Verzweigungsgrad, ihr Gehalt an tert.-Butylgruppen und ihre Uneinheitlichkeit, so dass das damit erzielte Eigenschaftsprofil für einen Einsatz in kosmetischen und pharmazeutischen Formulierungen noch verbesserungswürdig ist. So weisen die eingesetzten Oligomere beispielsweise einen deutlichen Eigengeruch auf, der an Terpene erinnert.

Aus der US-A 3,102,064 ist die Verwendung wässriger Alkenylbernsteinsäureanhydrid-Emulsionen, die mit Hilfe von kationischer Särke stabilisiert sind, als Masseleimungsmittel für Papier und Papierprodukte bekannt.

Aus der EP-A 0 593 075 ist ebenfalls bekannt, wässrige Emulsionen von Alkenylbernsteinsäureanhydriden, die durch Reaktion von Propylen- oder n-Butylenoligomeren mit Maleinsäureanhydrid erhältlich sind, als Leimungsmittel zu verwenden.

Gemäß der Lehre der EP-A 0 609 879 werden Ethylenoligomeren, die weniger als 5 Gew.-% Isomere mit 17 oder weniger Kohlenstoffatomen im Molekül und mindestens 95 Gew.-% Isomere mit mindestens 18 Kohlenstoffatomen im Molekül enthalten, mit Maleinsäureanhydrid zu Alkenylbernsteinsäureanhydriden umgesetzt. Bei der Oligomerisierung von Ethylen entstehen α-Olefine, die üblicherweise vor der Umsetzung mit Maleinsäureanhydrid isomerisiert werden müssen, damit eine innenliegende Doppelbindung vorliegt. Die daraus erhältlichen Umsetzungsprodukte mit Maleinsäureanhydrid werden als Leimungsmittel bei der Papierherstellung verwendet. Die bekannten Alkenylbernsteinsäureanhydride hydrolysieren jedoch relativ schnell.

Außerdem sind wässrige Leimungsmittelzusammensetzungen bekannt, die ein emulgiertes Alkenylbernsteinsäureanhydrid, ein oberflächenaktives Mittel und ein kationisches Polymer enthalten, vgl. US-A 4,657,947 und WO-A 2004/059081.

Aus DE-A 34 04 071 sind ebenfalls Papierleimmittel bekannt, die Alkenylbernsteinsäureanhydride enthalten. Diese werden durch Addition von Maleinsäureanhydrid an verzweigte innenständige Olefine mit 14 bis 36 Kohlenstoffatomen erhalten, welche durch Oligomerisierung eines Olefins oder eines Gemisches aus zwei oder mehreren Olefinen mit 6 bis 18 C-Atomen erhalten werden.

Der vorliegenden Erfindung liegt die Aufgabe zugrunde, neue Stoffe zur Verfügung zu stellen, die bei der Papierherstellung als Leimungsmittel einsetzbar sind und die gegenüber den bekannten Alkenylbernsteinsäureanhydriden vorteilhaftere anwendungstechnische Eigenschaften haben.

Die Aufgabe wird erfindungsgemäß gelöst mit Alkenylbernsteinsäureanhydriden, die durch Umsetzung von Oligomeren von C₄- bis G₆-Olefinen und Maleinsäureanhydrid herstellbar sind, wenn man bei der Umsetzung mit Maleinsäureanhydrid Mischungen von Oligomeren mit mindestens 12 C-Atomen einsetzt, die erhältlich sind durch Oligomerisierung eines Kohlenwasserstoffgemisches, das mindestens zwei Olefine mit 4 bis 8 C-Atomen enthält, an einem Katalysator, der ein Übergangsmetall enthält, und man bei der Oligomerisierung ein Kohlenwasserstoffgemisch einsetzt, das 20 bis 100 Gew.-% C₄-Olefine, 0 bis 80 Gew.-% C₅-Olefine, 0 bis 60 Gew.-% C₆-Olefine und 0 bis 10 Gew.-% von den zuvor genannten Olefinen verschiedene Olefine, jeweils bezogen auf den Gesamtolefingehalt, enthält.

Gegenstand der Erfindung ist außerdem ein Verfahren zur Herstellung von Alkenylbernsteinsäureanhydriden durch Umsetzung von Oligomeren von C₄- bis C₈-Olefinen und Maleinsäureanhydrid, wobei man bei der Umsetzung mit Maleinsäureanhydrid Mischungen von Oligomeren mit mindestens 12 C-Atomen einsetzt, die erhältlich sind durch Oligomerisierung eines Kohlenwasserstoffgemisches, das mindestens zwei Olefine mit 4 bis 8 C-Atomen enthält, an einem Katalysator, der ein Übergangsmetall enthält, und man bei der Oligomerisierung ein Kohlenwasserstoffgemisch einsetzt, das 20 bis 100 Gew.-% C₄-Olefine, 0 bis 80 Gew.-% C₅-Olefine, 0 bis 60 Gew.-% C₆-Olefine und 0 bis 10 Gew.-% von den zuvor genannten Olefinen verschiedene Olefine, jeweils bezogen auf den Gesamtolefingehalt, enthält.

Um die Oligomeren zu charakterisieren, kann man sie beispielsweise vollständig hydrieren, so dass sie keine Doppelbindungen mehr aufweisen. Als Hydrierungskatalysatoren können in der Regel alle Katalysatoren des Standes der Technik eingesetzt werden, die die Hydrierung von Olefinen zu den entsprechenden Alkanen katalysieren. Die Katalysatoren können sowohl in heterogener Phase als auch als Homogenkatalysatoren eingesetzt werden. Vorzugsweise enthalten die Hydrierungskatalysatoren wenigstens ein Metall der Gruppe VIII. Besonders geeignete Metalle der Gruppe VIII sind ausgewählt unter Ruthenium, Cobalt, Rhodium, Nickel, Palladium und Platin.

Die Metalle können auch als Gemische eingesetzt werden. Außerdem können die Katalysatoren neben den Metallen der Gruppe VIII auch geringe Mengen weiterer Metalle, beispielsweise Metalle der Gruppe VIIa, insbesondere Rhenium, oder Metalle der Gruppe Ib, d. h. Kupfer, Silber oder Gold, enthalten. Besonders bevorzugte Metalle der Gruppe VIII sind Ruthenium, Nickel, Palladium und Platin, insbesondere Platin, Nickel und Palladium, und stärker bevorzugt Palladium und Nickel. Speziell enthält der Katalysator Palladium als katalytisch aktive Spezies. Die Hydrierung erfolgt bei einer Temperatur von vorzugsweise 20 bis 250°C, besonders bevorzugt von 50 bis 240°C und insbesondere von 150 bis 220°C. Der Reaktionsdruck der Hydrierreaktion liegt vorzugsweise im Bereich von 1 bis 300 bar, besonders bevorzugt von 50 bis 250 bar und insbesondere von 150 bis 230 bar.

Bei der Hydrierung erhält man ein Isoalkangemisch, dessen ¹H-NMR-Spektrum im Bereich einer chemischen Verschiebung δ von 0,6 bis 1,0 ppm, bezogen auf Tetramethylsilan, ein Flächenintegral von 25 bis 70 %, bezogen auf die Gesamtintegralfläche, aufweist. Es hat vorzugsweise im ¹H-NMR-Spektrum im Bereich einer chemischen Verschiebung δ von 0,6 bis 1,0 ppm ein Flächenintegral von 30 bis 60 %, insbesondere von 35 bis 55 %, bezogen auf die Gesamtintegralfläche.

Des Weiteren bevorzugt weist das Isoalkangemisch im ¹H-NMR-Spektrum im Bereich einer chemischen Verschiebung δ von 0,5 bis 3 ppm (d. h. im Bereich der aliphatischen Protonen) ein Flächenintegral von bis zu 95 %, besonders bevorzugt von bis zu 98 %, bezogen auf die Gesamtintegralfläche, auf.

Solche Isoalkangemische weisen im Wesentlichen keine tert.-Butylgruppen (-C(CH₃)₃) auf. Der Anteil an endständigen tert.-Butylgruppen beträgt vorzugsweise höchstens 20 %, besonders bevorzugt höchstens 10 %, insbesondere höchstens 5 % und speziell höchstens 2 %.

Vorzugsweise sind die Isoalkane einheitlich aufgebaut. So weisen sie, bezogen auf die längste durchgängige Kohlenstoffkette, im Wesentlichen oder ausschließlich Methylverzweigungen auf. Der Anteil an Seitenketten mit Alkylgruppen, die 2 oder mehr als zwei Kohlenstoffatome aufweisen, beträgt weniger als 20 %, bevorzugt höchstens 10 %, besonders bevorzugt höchstens 5 %, insbesondere höchstens 1 %, bezogen auf die Gesamtzahl an Verzweigungsstellen.

Vorzugsweise enthalten die Isoalkangemische 50 bis 90 Gew.-%, insbesondere 60 bis 80 Gew.-% Alkane mit 16 Kohlenstoffatomen und 0 bis 30 Gew.-%, insbesondere 10 bis 20 Gew.-% Alkane mit 20 Kohlenstoffatomen.

Das Alkangemisch enthält vorzugsweise wenigstens 70 Gew.-%, bevorzugt wenigstens 85 Gew.-%, insbesondere wenigstens 95 Gew.-% Alkane mit einer geraden Kohlenstoffatomanzahl. Eine spezielle Ausführung ist ein Isoalkangemisch, das im Wesentlichen aus Alkanen mit 12 oder 16 Kohlenstoffatomen besteht.

Die Isoalkangemische weisen vorzugsweise einen Verzweigungsgrad V im Bereich von 0,1 bis 0,35, besonders bevorzugt 0,12 bis 0,3, insbesondere 0,15 bis 0,27 und speziell 0,17 bis 0,23 auf.

Die aus den Oligomermischungen durch Hydrieren erhältlichen Produkte haben beispielsweise einen Verzweigungsgrad V, molekulargewichtsunabhängig definiert als Zahl der Verzweigungen pro Kohlenstoffatom (V = Anzahl der Verzweigungen/Anzahl der Kohlenstoffatome, z. B. n-Octan: 0/8 = 0, Methylheptan: 1/8 = 0,125, Dimethylhexan: 2/8 = 0,25, Squalan (2,6,10,15,19,23-Hexamethyltetracosan): 6/30 = 0,2.

Die Oligomeren haben beispielsweise, jeweils bezogen pro 4 Kohlenstoffatome, vorzugsweise 0,7 bis 1,4, insbesondere 0,8 bis 1,3 CH₃-Gruppen und 0,7 bis 1,3, vorzugsweise 0,8 bis 1,2 und insbesondere 0,9 bis 1,1 Olefingruppen.

Geeignete Kohlenwasserstoff-Einsatzmaterialien für die Herstellung von OlefinOligomeren sind prinzipiell alle Verbindungen, die 4 bis 8 Kohlenstoffatome und wenigstens eine ethylenisch ungesättigte Doppelbindung enthalten. Vorzugsweise wird hierfür ein technisch zur Verfügung stehendes olefinhaltiges Kohlenwasserstoffgemisch eingesetzt.

Bevorzugte großtechnisch zur Verfügung stehende Olefingemische resultieren aus der Kohlenwasserstoff-Spaltung bei der Erdölverarbeitung, beispielsweise durch Katcracken, wie Fluid Catalytic Cracking (FCC), Thermocracken oder Hydrocracken mit anschließender Dehydrierung. Ein bevorzugtes technisches Olefingemisch ist der C₄-Schnitt. C₄-Schnitte sind beispielsweise durch Fluid Catalytic Cracking oder Steamcracken von Gasöl bzw. durch Steamcracken von Naphtha erhältlich. Je nach Zusammensetzung des C₄-Schnitts unterscheidet man den Gesamte-Schnitt (Roh-C₄-Schnitt), das nach der Abtrennung von 1,3-Butadien erhaltene so genannte Raffinat I sowie das nach der Isobutenabtrennung erhaltene Raffinat II. Ein weiteres geeignetes technisches Olefingemisch ist der bei der Naphtha-Spaltung erhältliche C₅-Schnitt.

Für die Oligomerisierung geeignete olefinhaltige Kohlenwasserstoffgemische mit 4 bis 8 Kohlenstoffatomen lassen sich weiterhin durch katalytische Dehydrierung geeigneter großtechnisch zur Verfügung stehender Paraffingemische erhalten. So gelingt beispielsweise die Herstellung von C₄-Olefin-Gemischen aus Flüssiggasen (liquified petroleum gas, LPG) und verflüssigbaren Erdgasen (liquified natural gas, LNG). Letztere umfassen neben der LPG-Fraktion auch zusätzlich größere Mengen höhermolekularer Kohlenwasserstoffe (leichtes Naphtha) und eignen sich somit auch zur Herstellung von C₅- und C₆-Olefin-Gemischen. Die Herstellung von olefinhaltigen Kohlenwasserstoffgemischen, die Monoolefine mit 4 bis 6 Kohlenstoffatomen enthalten, aus LPG- oder LNG-Strömen gelingt nach üblichen, dem Fachmann bekannten Verfahren, die neben der Dehydrierung in der Regel noch einen oder mehrere Aufarbeitungsschritte umfassen. Dazu zählt beispielsweise die Abtrennung wenigstens eines Teils der in den zuvor genannten Olefin-Einsatzgemischen enthaltenen gesättigten Kohlenwasserstoffe. Diese können beispielsweise erneut zur Herstellung von Olefin-Einsatzmaterialien durch Crackung und/oder Dehydrierung eingesetzt werden. Die für die Oligomerisierung eingesetzten Olefine können jedoch auch einen Anteil gesättigter Kohlenwasserstoffe enthalten, die sich gegenüber den Oligomerisierungsbedingungen inert verhalten. Der Anteil dieser gesättigten Komponenten beträgt im Allgemeinen höchstens 60 Gew.-%, bevorzugt höchstens 40 Gew.-%, besonders bevorzugt höchstens 20 Gew.-%, bezogen auf die Gesamtmenge der in dem Kohlenwasserstoff-Einsatzmaterial enthaltenen Olefine und gesättigten Kohlenwasserstoffe.

Erfindungsgemäß wird für die Oligomerisierung ein Kohlenwasserstoffgemisch bereitgestellt, das 20 bis 100 Gew.-% C₄-Olefine, 0 bis 80 Gew.-% C₅-Olefine, 0 bis 60 Gew.-% C₆-Olefine und 0 bis 10 Gew.-% von den zuvor genannten Olefinen verschiedene Olefine, jeweils bezogen auf den Gesamtolefingehalt, enthält.

Vorzugsweise wird für die Oligomerisierung ein Kohlenwasserstoffgemisch bereitgestellt, das einen Gehalt an linearen Monoolefinen von mindestens 80 Gew.-%, besonders bevorzugt mindestens 90 Gew.-% und insbesondere mindestens 95 Gew.-%, bezogen auf den Gesamtolefingehalt, aufweist. Dabei sind die linearen Monoolefine ausgewählt unter 1-Buten, 2-Buten, 1-Penten, 2-Penten, 1-Hexen, 2-Hexen, 3-Hexen und Mischungen davon. Es kann von Vorteil sein, wenn das zur Oligomerisierung eingesetzte Kohlenwasserstoffgemisch bis zu 20 Gew.-%, bevorzugt bis zu 5 Gew.-%, insbesondere bis zu 3 Gew.-% verzweigte Olefine, bezogen auf den Gesamtolefingehalt, enthält.

Besonders bevorzugte Oligomere erhält man, wenn man zur ihrer Herstellung ein C₄-Olefingemisch einsetzt. Der Butengehalt, bezogen auf 1-Buten, 2-Buten und Isobuten, des C₄-Olefingemisches beträgt vorzugsweise 10 bis 100 Gew.-%, besonders bevorzugt 50 bis 99 Gew.-%, und insbesondere 70 bis 95 Gew.-%, bezogen auf den Gesamtolefingehalt. Vorzugsweise liegt das Verhältnis von 1-Buten zu 2-Buten in einem Bereich von 20 : 1 bis 1 : 2, insbesondere bei etwa 10 : 1 bis 1 : 1. Vorzugsweise enthält das C₄-Kohlenwasserstoffgemisch weniger als 5 Gew.-%, insbesondere weniger als 3 Gew.-% Isobuten. Aus solchen Gemischen werden Oligomergemische hergestellt und daraus beispielsweise Trimere, Tetramere, Pentamere und/oder Hexamere isoliert.

Die Bereitstellung der olefinhaltigen Kohlenwasserstoffe kann eine Abtrennung von verzweigten Olefinen umfassen. Geeignet sind übliche, aus dem Stand der Technik bekannte Abtrennverfahren, die auf unterschiedlichen physikalischen Eigenschaften von linearen und verzweigten Olefinen bzw. auf unterschiedlichen Reaktivitäten, die selektive Umsetzungen ermöglichen, beruhen. So kann beispielsweise Isobuten von C₄-Olefingemischen, wie Raffinat I, durch eine der folgenden Methoden abgetrennt werden:
- Molsiebtrennung, .
- fraktionierte Destillation,
- reversible Hydratisierung zu tert.-Butanol,
- sauer katalysierte Alkohol-Addition an einen tertiären Ether, z. B. Methanol-Addition zu Methyl-tert.-butylether (MTBE),
- irreversible katalysierte Oligomerisierung zu Di- und Tri-Isobuten,
- irreversible Polymerisation zu Polyisobuten,

Derartige Verfahren sind in K. Weissermel, H.-J. Arpe, Industrielle organische Chemie, 4. Auflage, S. 76 - 81, VCH-Verlagsgesellschaft Weinheim, 1994 beschrieben, worauf hier in vollem Umfang Bezug genommen wird.

Vorzugsweise wird bei der Oligomerisierung ein Raffinat II eingesetzt., das beispielsweise die folgende Zusammensetzung hat:

| | |
|---|---|
| 0,5 bis 5 Gew.-% | Isobutan, |
| 5 bis 20 Gew.-% | n-Butan, |
| 20 bis 40 Gew.-% | trans-2-Buten, |
| 10 bis 20 Gew.-% | cis-2-Buten, |
| 25 bis 55 Gew.-% | 1-Buten, |
| 0,5 bis 5 Gew.-% | Isobuten |

sowie Spurengase, wie 1,3-Butadien, Propen, Propan, Cyclopropan, Propadien, Methylcyclopropan, Vinylacetylen, Pentene, Pentane etc. im Bereich von jeweils maximal 1 Gew.-%. Aus dem oben angegebenen Gemisch werden vorzugsweise Tetramere hergestellt.

Ein geeignetes Raffinat II weist z.B. folgende typische Zusammensetzung auf:

| | |
|---|---|
| i-, n-Butan | 26 Gew.-% |
| i-Buten | 1 Gew.-% |
| 1-Buten | 26 Gew.-% |
| trans-2-Buten | 31 Gew.-% |
| cis-2-Buten | 16 Gew.-%. |

Es eignet sich insbesondere zur Herstellung von Tetrameren. Sind Diolefine oder Alkine in dem olefinreichen Kohlenwasserstoffgemisch vorhanden, so können diese vor der Oligomerisierung auf vorzugsweise weniger als 10 Gew.-ppm aus demselben entfernt werden. Sie werden bevorzugt durch selektive Hydrierung, z. B. gemäß EP-A 81 041 und DE-A 15 68 542 entfernt, besonders bevorzugt durch eine selektive Hydrierung bis auf einen Restgehalt von unter 5 Gew.-ppm, insbesondere 1 Gew.-ppm.

Aus dem olefinreichen Kohlenwasserstoffgemisch werden zweckmäßigerweise außerdem sauerstoffhaltige Verbindungen, wie Alkohole, Aldehyde, Ketone oder Ether, weitgehend entfernt. Hierzu kann das olefinreiche Kohlenwasserstoffgemisch mit Vorteil über ein Adsorptionsmittel, wie z. B. ein Molekularsieb, insbesondere eines mit einem Porendurchmesser von > 4 Å bis 5 Å, geleitet werden. Die Konzentration an sauerstoffhaltigen, schwefelhaltigen, stickstoffhaltigen und halogenhaltigen Verbindungen im olefinreichen Kohlenwasserstoffgemisch beträgt vorzugsweise weniger als 1 Gew.-ppm, insbesondere weniger als 0,5 Gew.-ppm.

### Katalysatoren für die Oligomerisierung

Im Rahmen der vorliegenden Erfindung umfasst der Begriff "Oligomere" Dimere, Trimere, Tetramere und höhere Produkte aus der Aufbaureaktion der eingesetzten Olefine. Die Oligomeren sollen mindestens 12 C-Atome, vorzugsweise 12 bis 24 C-Atome, insbesondere 16 bis 20 C-Atome im Molekül enthalten. Vorzugsweise sind die Mischungen von Oligomeren ausgewählt unter Dimeren, insbesondere von C₆- bis C₈-Olefinen, Trimeren, insbesondere von C₄- bis C₆-Olefinen und Tetrameren, insbesondere von C₄-Olefinen. Die Mischungen der Oligomeren sind ihrerseits olefinisch ungesättigt. Durch geeignete Wahl des zur Oligomerisierung eingesetzten Kohlenwasserstoffeinsatzmaterials und des Oligomerisierungskatalysators, wie im Folgenden beschrieben, können die angestrebten Mischungen von Oligomeren erhalten werden.

Zur Oligomerisierung kann ein Reaktionssystem eingesetzt werden, das einen oder mehrere, gleiche oder verschiedene Reaktoren umfasst. Im einfachsten Fall wird zur Oligomerisierung ein einzelner Reaktor eingesetzt. Es können jedoch auch mehrere Reaktoren eingesetzt werden, die jeweils gleiche oder verschiedene Vermischungscharakteristiken aufweisen. Die einzelnen Reaktoren können gewünschtenfalls durch Einbauten ein- oder mehrfach unterteilt sein. Bilden zwei oder mehrere Reaktoren das Reaktionssystem, so können diese untereinander beliebig verschaltet sein, z. B. parallel oder in Reihe. In einer geeigneten Ausführung wird z.B. ein Reaktionssystem eingesetzt, das aus zwei in Reihe geschalteten Reaktoren besteht.

Geeignete druckfeste Reaktionsapparaturen für die Oligomerisierung sind dem Fachmann bekannt. Dazu zählen die allgemein üblichen Reaktoren für Gas-fest- und Gasflüssig-Reaktionen, wie z. B. Rohrreaktoren, Rührkessel, Gasumlaufreaktoren, Blasensäulen etc., die gegebenenfalls durch Einbauten unterteilt sein können. Vorzugsweise werden Rohrbündelreaktoren oder Schachtöfen eingesetzt. Wird zur Oligomerisierung ein heterogener Katalysator eingesetzt, so kann dieser in einem einzigen oder in mehreren Katalysator-Festbetten angeordnet sein. Dabei ist es möglich, in verschiedenen Reaktionszonen unterschiedliche Katalysatoren einzusetzen. Bevorzugt ist jedoch der Einsatz des gleichen Katalysators in allen Reaktionszonen.

Die Temperatur bei der Oligomerisierungsreaktion liegt im Allgemeinen in einem Bereich von etwa 20 bis 280 °C, bevorzugt von 25 bis 200 °C, insbesondere von 30 bis 140 °C. Der Druck bei der Oligomerisierung liegt im Allgemeinen in einem Bereich von etwa 1 bis 300 bar, vorzugsweise von 5 bis 100 bar und insbesondere von 20 bis 70 bar. Umfasst das Reaktionssystem mehr als einen Reaktor, so können diese gleiche oder verschiedene Temperaturen und gleiche oder verschiedene Drücke aufweisen. So kann beispielsweise im zweiten Reaktor einer Reaktorkaskade eine höhere Temperatur und/oder ein höherer Druck als im ersten Reaktor eingestellt werden, z. B. um einen möglichst vollständigen Umsatz zu erzielen.

In einer speziellen Ausführung werden die zur Oligomerisierung eingesetzten Temperatur und Druckwerte so gewählt, dass das olefinhaltige Einsatzmaterial flüssig oder im überkritischen Zustand vorliegt.

Die Oligomerisierung wird vorzugsweise adiabatisch durchgeführt. Dieser Begriff wird im Rahmen der vorliegenden Erfindung im technischen und nicht im physikochemischen Sinne verstanden. So verläuft die Oligomerisierungsreaktion in der Regel exotherm, so dass das Reaktionsgemisch beim Strömen durch das Reaktionssystem, beispielsweise ein Katalysatorbett, eine Temperaturerhöhung erfährt. Unter adiabatischer Reaktionsführung wird eine Vorgehensweise verstanden, bei der die in einer exothermen Reaktion freiwerdende Wärmemenge von der Reaktionsmischung im Reaktor aufgenommen und keine Kühlung durch Kühlvorrichtungen angewandt wird. Somit wird die Reaktionswärme mit dem Reaktionsgemisch aus dem Reaktor abgeführt, abgesehen von einem Restanteil, der durch natürliche Wärmeleitung und Wärmeabstrahlung vom Reaktor an die Umgebung abgegeben wird.

Zur Oligomerisierung wird ein Übergangsmetalle enthaltender Katalysator eingesetzt. Bevorzugt handelt es sich dabei um heterogene Katalysatoren. Bevorzugte Katalysatoren, die bekanntermaßen eine geringe Oligomeren-Verzweigung bewirken, sind Nickel enthaltende Katalysatoren. Solche Katalysatoren, die bekanntermaßen eine geringe Oligomeren-Verzweigung bewirken, sind dem Fachmann allgemein bekannt. Dazu zählen die in Catalysis Today, 6, 329 (1990), insbesondere Seiten 336-338, sowie die in der DE-A 43 39 713 (= WO-A 95/14647) und der DE-A 199 57 173 beschriebenen Katalysatoren, auf die hiermit ausdrücklich Bezug genommen wird. Ein geeignetes Oligomerisierungsverfahren, bei dem der zur Oligomerisierung eingesetzten Feed-Strom aufgeteilt und mindestens zwei bei unterschiedlichen Temperaturen betriebenen Reaktionszonen zugeführt wird ist in der EP-A 1 457 475 beschrieben, auf die ebenfalls Bezug genommen wird.

Die eingesetzten heterogenen Nickel enthaltenden Katalysatoren können unterschiedliche Strukturen aufweisen. Geeignet sind prinzipiell Vollkatalysatoren sowie geträgerte Katalysatoren. Letztere werden bevorzugt eingesetzt. Die Trägermaterialien können z. B. Kieselsäure, Tonerde, Aluminosilicate, Aluminosilicate mit Schichtstrukturen und Zeolithe, wie Mordenit, Faujasit, Zeolith X, Zeolith-Y und ZSM-5, Zirkoniumoxid, das mit Säuren behandelt ist, oder sulfatiertes Titandioxid sein. Besonders geeignet sind Fällungskatalysatoren, die durch Mischen wässriger Lösungen von Nickelsalzen und Silicaten, z. B. Natriumsilicat mit Nickelnitrat, und gegebenenfalls Aluminiumsalzen, wie Aluminiumnitrat, und Calcinieren erhältlich sind. Weiterhin sind Katalysatoren verwendbar, die durch Einlagerung von Ni²⁺-Ionen durch Ionenaustausch in natürliche oder synthetische Schichtsilicate, wie Montmorillonite, erhalten werden. Geeignete Katalysatoren können auch durch Imprägnieren von Kieselsäure, Tonerde oder Alumosilicaten mit wässrigen Lösungen löslicher Nickelsalze, wie Nickelnitrat, Nickelsulfat oder Nickelchlorid, und anschließende Calcinierung erhalten werden.

Nickeloxid enthaltende Katalysatoren sind bevorzugt. Besonders bevorzugt sind Katalysatoren, die im Wesentlichen aus NiO, SiO₂, TiO₂ und/oder ZrO₂ sowie gegebenenfalls Al₂O₃ bestehen. Am meisten bevorzugt ist ein Katalysator, der als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumdioxid enthält. Ein solcher Katalysator ist beispielsweise durch Fällung einer Katalysatormasse bei pH 5 bis 9 durch Zugabe einer Nickelnitrat enthaltenden wässrigen Lösung zu einer Alkaliwasserglaslösung, die Titandioxid und/oder Zirkondioxid enthält, Filtrieren, Trocknen und Tempern bei 350 bis 650 °C erhältlich. Zur Herstellung dieser Katalysatoren wird im Einzelnen auf die DE-A 43 39 713 verwiesen. Auf die Offenbarung dieser Druckschrift und den darin zitierten Stand der Technik wird vollinhaltlich Bezug genommen.

In einer weiteren Ausführungsform wird als Katalysator für die Oligomerisierung ein Nickelkatalysator gemäß der DE-A 199 57 173 eingesetzt. Dabei handelt es sich im Wesentlichen um Aluminiumoxid, das mit einer Nickelverbindung und einer Schwefelverbindung beaufschlagt wurde. Vorzugsweise liegt im fertigen Katalysator ein molares Verhältnis von Schwefel zu Nickel im Bereich von 0,25 : 1 bis 0,38 : 1 vor.

Der Katalysator liegt vorzugsweise in stückiger Form, z. B. in Form von Tabletten, z. B. mit einem Durchmesser von 2 bis 6 mm und einer Höhe von 3 bis 5 mm, Ringen mit z. B. 5 bis 7 mm Außendurchmesser, 2 bis 5 mm Höhe und 2 bis 3 mm Lochdurchmesser, oder Strängen unterschiedlicher Länge eines Durchmessers von z. B. 1,5 bis 5 mm, vor. Derartige Formen werden auf an sich bekannte Weise durch Tablettierung oder Extrusion, meist unter Verwendung eines Tablettierhilfsmittels, wie Graphit oder Stearinsäure, erhalten.

Zur Auftrennung kann das Reaktionsgemisch der Oligomerisierung einem oder mehreren Trennschritten unterworfen werden. Geeignete Trennvorrichtungen sind die üblichen, dem Fachmann bekannten Apparaturen. Dazu zählen z. B. Destillationskolonnen, z. B. Bodenkolonnen, die gewünschtenfalls mit Glocken, Siebplatten, Siebböden, Ventilen, Seitenabzügen, etc. ausgerüstet sein können, Verdampfer, wie Dünnschichtverdampfer, Fallfilmverdampfer, Wischblattverdampfer, Sambay-Verdampfer, etc. und Kombinationen davon. Bevorzugt erfolgt die Isolierung der Olefinfraktion durch ein- oder mehrstufige fraktionierte Destillation.

### Reaktion der Oligomeren mit Maleinsäureanhydrid

Die oben beschriebenen Mischungen von Oligomeren werden in Analogie zu bekannten Verfahren mit Maleinsäureanhydrid umgesetzt. Dabei entstehen Mischungen von Alkenylbernsteinsäureanhydriden. Gesättigte Kohlenwasserstoffe, die gegebenenfalls in der Mischung der Oligomeren enthalten sind, stören bei der Umsetzung mit Maleinsäureanhydrid in aller Regel nicht. Man kann sie jedoch auch vor der Umsetzung z.B. durch Destillation, aus dem Oligomerengemisch entfernen. Die Umsetzung mit Maleinsäureanhydrid erfolgt vorzugsweise in Substanz bei einer Temperatur in dem Bereich von beispielsweise 100 bis 280°C, vorzugsweise bei 150 bis 250°C meistens bei 180 bis 230°C. Die Umsetzung wird vorzugsweise in druckdichten Apparaturen wie Autoklaven, durchgeführt. Sie kann diskontinuierlich oder kontinuierlich erfolgen. Die Verweilzeit der Reaktionsmischung in der Reaktionszone hängt von der jeweils gewählten Reaktionstemperatur ab. Höhere Temperaturen erfordern kürzere Reaktionszeiten als tiefere Temperaturen. So kann die Reaktionszeit beispielsweise 5 Sekunden bis 10 Stunden betragen. Pro Mol Oligomer in der Mischung von Oligomeren setzt man beispielsweise 0,2 bis 5 Mol, vorzugsweise 0,3 bis 3 Mol und insbesondere 0,8 bis 2,4 Mol Maleinsäureanhydrid ein. Die bei der Umsetzung entstehenden Mischungen von Alkenylbernsteinsäureester können ohne einen zusätzlichen Reinigungsschritt als Leimungsmittel für Papier verwendet werden. Man kann jedoch auch vorher niedrigsiedende Anteile aus dem Reaktionsgemisch abdestillieren oder in einer vorteilhaften Ausführungsform der Erfindung das Reaktionsgemisch fraktioniert destillieren. Die Hauptmenge der Mischung aus Alkenylbernsteinsäureanhydriden wird dabei unter einem Druck von 1 mbar in dem Temperaturbereich von 180 bis 240°C aus der Mischung abdestilliert. Meistens beendet man die Destillation zu einem Zeitpunkt, an dem die unter einem Druck von 1 mbar übergehenden Dämpfe eine Temperatur von 230°C haben.

### Verwendung der Alkenylbernsteinsäureanhydride

Gegenstand der Erfindung ist außerdem die Verwendung der oben beschriebenen Mischungen von Alkenylbernsteinsäureanhydriden als Leimungsmittel für Papier. Die Mischungen der Alkenylbernsteinsäureanhydride werden zu diesem Zweck in Wasser in Gegenwart mindestens eines Schutzkolloids emulgiert. Als Schutzkolloid eignen sich beispielsweise alle Sorten von Stärke, z.B. sowohl Amylose als auch Amylopektin, native Stärken, hydrophob oder hydrophil modifizierte Stärken, abgebaute Stärken, wobei der Stärkeabbau beispielsweise oxidativ, thermisch, hydrolytisch oder enzymatisch vorgenommen werden kann und wobei für den Stärkeabbau sowohl native als auch modifizierte Stärken eingesetzt werden können, Dextrine und vernetzte, wasserlösliche Stärken, vgl. Ullmanns Encyclopedia of Industrial Chemistry, 6. Auflage, Band 33, unter Stärke, Seiten 735 bis 737. Übliche Vernetzer für die Herstellung solcher Stärken sind z.b. POCl₃, Epichlorhydrin und gemischte Anhydride. Weitere Beispiele für Schutzkolloide sind Glykogene, Inuline, Chitine, Chitosane, Pektine, wasserlösliche Cellulosederivate wie Carboxyalkylcellulosen, Cellulosesulfat, Cellulosephosphorsäureester, Cellu-Ioseformiat, Hydroxyethylcellulosen, Hemicellulosen wie Xylane, Mannane, Galactane, Glycoproteine und Mucopolysaccharide.

Als Schutzkolloid werden vorzugsweise native Stärken eingesetzt, die beispielsweise mit Hilfe eines Stärkeaufschlusses in eine wasserlösliche Form überführt werden können, kationische Stärke, vorzugsweise kationisch modifizierte Kartoffelstärke sowie anionische modifizierte Stärken wie oxidierte Kartoffelstärke. Zu den bevorzugt eingesetzten Schutzkolloiden gehören auch anionisch modifizierte Stärken, die einem Molekulargewichtsabbau unterworfen wurden. Der Molekulargewichtsabbau wird vorzugsweise enzymatisch durchgeführt. Die mittlere Molmasse der abgebauten Stärken beträgt z.B. 500 bis 100 000, meistens 1000 bis 30 000. Geeignete abgebaute Stärken werden beispielsweise in der EP-A 0 257 412 und in der EP-A 0 276 770 beschrieben.

Als Schutzkolloide kommen auch Kondensationsprodukte aus
Naphthalinsulfonsäure und Formaldehyd,
Phenol, Phenolsulfonsäure und Formaldehyd,
Naphthalinsulfonsäure, Formaldehyd und Harnstoff sowie
Phenol, Phenolsulfonsäure, Formaldehyd und Harnstoff

in Betracht. Es handelt sich hierbei um bekannte Verbindungen, die beispielsweise durch Kondensation der obengenannten Bestandteile in Gegenwart von Säuren wie Schwefelsäure oder p-Toluolsulfonsäure als Katalysator herstellbar sind. Anstelle der freien Säuren kann man auch die Salze der Naphthalinsulfonsäure oder der Phenolsulfonsäure bei der Kondensation einsetzen. Das Molverhältnis der zuletzt genannten Säuren zu Formaldehyd beträgt bei der Kondensation z.B. 1 : 0,1 bis 1 : 2, meistens 1 : 0,5 bis 1 : 1. Falls die Kondensation von Naphthalinsulfonsäure und von Phenolsulfonsäure mit Formaldehyd noch zusätzlich in Gegenwart von Harnstoff durchgeführt wird, setzt man beispielsweise, bezogen auf ein Mol der Mischung aus Phenol und Phenolsulfonsäure bzw. auf 1 Mol Naphthalinsulfonsäure 0,1 bis 5 Mol Harnstoff ein.

Weitere geeignete Schutzkolloide sind Polymere von ethylenisch ungesättigten C₃- bis C₅-Carbonsäuren, insbesondere Polymere der Acrylsäure, sowie amphiphile Copolymerisate aus (i) hydrophoben monoethylenisch ungesättigten Monomeren und (ii) monoethylenisch ungesättigten Carbonsäuren, monoethylenisch ungesättigten Sulfonsäuren, monoethylenisch ungesättigten Phosphonsäuren oder deren Mischungen. Ein Beispiel für derartige Schutzkolloide sind Copolymere aus (i) Styrol, Isobuten und/oder Diisobuten und (ii) einer ethylenisch ungesättigten C₃- bis C₅-Carbonsäure wie Acrylsäure, Maleinsäure und/oder Methacrylsäure. Die anionischen Schutzkolloide können in Form der freien Säuren sowie in partiell oder vollständig neutralisierter Form verwendet werden. Als Neutralisationsmittel kommen z.B. Alkalilaugen, Ammoniak und Amine sowie Erdalkalimetallbasen in Betracht. Meistens setzt man zur Neutralisation Natronlauge, Soda, Natriumhydrogencarbonat, Ammoniak, Triethanolamin, Morpholin, Magnesiumoxid oder Calciumhydroxid ein. Die Molmasse M_{w} der amphiphlen Copolymerisate und der Homopolymerisate der ethylenisch ungesättigten Carbonsäuren liegt beispielsweise in de Bereich von 500 bis 100 000, vorzugsweise 1000 bis 10 000.

Als Schutzkolloide können prinzipiell alle oberflächenaktiven Verbindungen bzw. Polymere eingesetzt werden, die gemäß dem Stand der Technik in Alkenylbernsteinsäureanhydrid-Emulsionen enthalten sind, vgl. US-A 4,657,946 und WO-A 2004/059081. So eignen sich insbesondere die aus der US-A 4,657,946 bekannten kationischen Polymeren mit Molmassen von 10 000 bis weniger als 1 Million in Verbindung mit oberflächenaktiven Mitteln. Als kationische Polymere kommen Diallyldimethylammoniumchlorid, basische Acrylate und basische Methacrylate in Form der freien Basen oder der quaternierten Produkte in Betracht, z.B. Dimethylaminoethylmethacrylat, Dimethylaminoethylacrylat, Diethylaminoethylacrylat, Diethylaminoethylmethacrylat und die entsprechenden quaternierten Produkte. Als Quaternierungsmittel kann man beispielsweise Methylchlorid, Ethylchlorid, Hexylchlorid, Benzylchlorid oder Dimethylsulfat verwenden. Als kationische Polymere eignen sich außerdem Mannichprodukte aus Acrylamid, Formaldehyd und einem sekundären Amin sowie quaternierte Mannichprodukte. Bevorzugte Polymere aus dieser Gruppe sind Diallyldimethylammoniumchlorid sowie basische (Meth)Acrylate in Form der Salze oder der quaternierten Verbindungen. Kationische Polymere, die als Schutzkolloid für Alkenylbernsteinsäureanhydride in Betracht kommen werden ausführlich in der US-A 4,657,946, Spalte 3, Zeilen 56 bis Spalte 5, Zeile 36 beschreiben. Beispiele für oberflächenaktive Verbindungen findet man in dieser Literaturstelle in Spalte 2, Zeilen 57 bis Spalte 3, Zeile 55.

Gebräuchliche oberflächenaktive Mittel für Alkenylbernsteinsäureanhydride werden beispielsweise in der zum Stand der Technik zitierten WO-A 2004/059081, Seite 11, Zeile 5 bis Seite 12, Zeile 16 und in der Tabelle auf Seite 31 beschrieben. Hierbei handelt es sich z.B. um Sulfosuccinate, Alkyl- und Arylamide, primäre, sekundäre und tertiäre Amine sowie die entsprechenden quaternären Salze, ethoxylierte Fettsäuren, Fettalkohole, ethoxylierte Fettalkohole, Fettsäureester, ethoxylierte Fettsäureester, Phosphatester, Polyethylenglykole, Alkylsulfonate, Arylsulfonate, Arylsulfate und Alkylsulfate.

Häufig eingesetzte Emulgiermittel sind grenzflächenaktive Stoffe wie Natrium-C₁₂- bis C₂₂-alkylsulfonate oder Polyvinyalkohol.

Die anionischen Schutzkolloide werden beispielsweise in einer Menge von 0,05 bis 20, vorzugsweise 0,5 bis 10 Gew.-%, bezogen auf die Mischung als Alkenylbernsteinsäureanhydriden, beim Emulgieren eingesetzt. Die amphiphilen Polymeren werden vorzugsweise in einer Menge von 0,1 bis 2 Gew.-%, bezogen auf die Mischung aus Alkenylbernsteinsäureanhydriden, verwendet. Bei den Schutzkolloiden auf Basis von Stärke und deren Derivaten setzt man beispielsweise auf 1 Gew.-Teil der Mischung aus Alkenylbernsteinsäureanhydriden 1 bis 10, vorzugsweise 2 bis 4 Gew.-Teile Stärke oder deren Derivate ein.

Die Mischungen aus Alkenylbernsteinsäurenanhydriden werden nach bekannten Verfahren unter Einwirkung von Scherkräften in Wasser in Gegenwart mindestens eines Schutzkolloids emulgiert. Das Emulgieren geschieht beispielsweise mit Hilfe von Hochdruckhomogenisatoren, Rotor-Stator-Apparaten oder durch Einwirkung von Ultraschall. Angaben über geeignete Vorrichtungen können beispielsweise der Veröffentlichung von H. Schubert et.al., Mischen und Rühren - Grundlagen und moderne Verfahren für die Praxis, VDI-Tagung, 23.24.11.1988, Baden-Baden, unter Neue Entwicklungen auf dem Gebiet der Emulgiertechnik, entnommen werden. Das Emulgieren erfolgt beispielsweise in dem Temperaturbereich von 0 bis 100, meistens von 20 bis 60 °C.

Die Herstellung der Emulsionen erfolgt in der Regel kurze Zeit vor der Anwendung, weil die Alkenylbernsteinsäureanhydride (ASA) in Gegenwart von Wasser hydrolysieren. Man stellt meistens zunächst konzentrierte wässrige Emulsionen von ASA her, z.B. beträgt die ASA-Konzentration bis zu 50 Gew.-%, vorzugsweise 10 bis 20 Gew.-%, und verdünnt danach die konzentrierten ASA-Emulsionen auf einen ASA-Gehalt von beispielsweise 0,7 bis 1,2 Gew.-%, vorzugsweise etwa 1 Gew.-%. Die verdünnten ASA-Emulsionen werden dann als Leimungsmittel für Papier eingesetzt.

Von besonderem technischen Interesse ist die Verwendung einer Mischung von Alkenylbernsteinsäureanhydriden, die erhältlich ist durch
(i) Oligomerisierung eines Kohlenwasserstoffgemisches, das

| | |
|---|---|
| 0,5 bis 5 Gew.-% | Isobutan, |
| 5 bis 20 Gew.-% | n-Butan, |
| 20 bis 40 Gew.-% | trans-2-Buten, |
| 10 bis 20 Gew.-% | cis-2-Buten, |
| 25 bis 55 Gew.-% | 1-Buten und |
| 0,5 bis 5 Gew.-% | Isobuten |

enthält zu einem Isobutan und n-Butan enthaltendem Gemisch von Oligomeren und
(ii) Umsetzung dieses Gemisches mit Maleinsäureanhydrid zu einer Mischung von C₁₆- bis C₂₄-Alkenylbernsteinsäureanhydriden, als Leimungsmittel für Papier, Pappe und Karton. Solche Leimungsmittel sind einfacher herstellbar als die aus dem Stand der Technik bekannten Umsetzungsprodukte eines n-Buten-Öligomers mit Maleinsäureanhydrid, weil man beispielsweise die Butenmischung ohne vorherige Isomerisierung direkt mit Maleinsäureanhydrid umsetzen kann. Aufgrund des hohen Verzweigungsgrades der Olefinmischung erhält man Umsetzungsprodukte mit Maleinsäureanhydrid, die einen niedrigen Schmelzpunkt aufweisen und die beispielsweise bei Raumtemperatur flüssig und dadurch leichter handhabbar sind.

Die Alkenylbernsteinsäureanhydride werden vorzugsweise als Masseleimungsmittel bei der Papierherstellung eingesetzt, können jedoch auch als Oberflächenleimungsmittel verwendet werden. Die dem Papierstoff zugesetzten Mengen an Leimungsmittel betragen beispielsweise 0,1 bis 2, vorzugsweise 0,5 bis 1,0 kg/t trockenes Papier.

Für die Herstellung von Papier, Pappe und Karton kann man von Cellulosefasern aller Art ausgehen, sowohl von natürlichen wie auch von zurückgewonnenen Fasern, insbesondere von Fasern aus Altpapier. Als Faserstoffe zur Herstellung der Pulpen kommen sämtliche dafür gebräuchlichen Qualitäten in Betracht, z.B. Holzstoff, gebleichter und ungebleichter Zellstoff sowie Papierstoffe aus allen Einjahrespflanzen. Zu Holzstoff gehören beispielsweise Holzschliff, thermomechanischer Stoff (TMP), chemothermomechanischer Stoff (CTMP), Druckschliff, Halbzellstoff, Hochausbeute-Zellstoff und Refiner Mechanical Pulp (RMP). Als Zellstoff kommen beispielsweise Sulfat-, Sulfit- und Natronzellstoffe in Betracht. Vorzugsweise verwendet man ungebleichten Zellstoff, der auch als ungebleichter Kraftzellstoff bezeichnet wird. Geeignete Einjahrespflanzen zur Herstellung von Papierstoffen sind beispielsweise Reis, Weizen, Zuckerrohr und Kenaf. Zur Herstellung der Pulpen kann auch mit Vorteil Altpapier verwendet werden, das entweder allein oder in Mischung mit anderen Faserstoffen eingesetzt wird oder man geht von Fasermischungen aus einem Primärstoff und zurückgeführtem gestrichenem Ausschuß aus, z. B. gebleichtes Kiefernsulfat in Mischung mit zurückgeführtem gestrichenem Ausschuß.

Die Alkenylbernsteinsäureanhydride werden vorzugsweise zum Leimen von Papierprodukten für die Herstellung von Flüssigverpackungen und von Papierprodukten verwendet, die im Bausektor benötigt werden z. B. geleimte Papiere bzw. Karton für Gipskartonplatten. Sie können außerdem bei der Herstellung von Liner, holzhaltigen und holzfreien Druck- und Schreibpapieren sowie Recyclingpapieren als Leimungsmittel verwendet werden.

Die Alkenylbernsteinsäureanhydride können außerdem als Härtungsmittel für Epoxidharze, als Additive für Kraftstoffe und Schmierstoffe (z.B. als Dispergiermittel), als Rost- und Korrosionsschutzmittel, als Tenside, als Dispergiermittel, insbesondere bei der Erdölförderung, als Nahrungsmitteladditive, für die Hydrophobierung von Leder und Textilien.
Die Alkenylbernsteinsäureanhydride können des weiteren zu Amid-, Imid- und Ester-Derivaten sowie zur Alkenylbernsteinsäure umgesetzt werden und dann als Dispergiermittel, als Tenside, als Additive für Schmieröle und als Antikorrosionsmittel eingesetzt werden.

Die in den Beispielen angegebenen Teile sind Gewichtsteile, die Prozentangaben bedeuten Gewichtsprozent, sofern aus dem Zusammenhang nichts anderes hervorgeht. Die Bestimmung des Cobb-Wertes erfolgte nach DIN 53 132 durch Lagerung der Papierblätter für einen Zeitraum von 60 Sekunden in Wasser. Die Wasseraufnahme wird in g/m² angegeben. Die Tintenschwimmzeit wurde gemäß DIN 53126 mit einer blauen Prüftinte bestimmt.

### Beispiele

Für die Herstellung einer Mischung von Oligomeren wurde folgendes Kohlenwasserstoffgemisch (Raffinat II) eingesetzt:
5 Teile Isobutan,
16 Teile n-Butan,
31 Teile 1-Buten,
28 Teile trans-2-Buten,
15 Teile cis-2-Buten und
2 Teile Isobuten.

Als Katalysator diente ein Material, das gemäß DE 4339713 zu 5*5mm Volltabletten verformt wurde (Zusammensetzung in Gew.%: 50% NiO, 12,5% TiO₂ 33,5% SiO₂, 4% Al₂O₃).

Die Versuche wurden in einer Reaktorkaskade, bestehend aus zwei hintereinandergeschalteten Reaktoren (Durchmesser 80 mm, Länge 4000mm, Zwischenkühlung zwischen den beiden Reaktoren) mit anschließender Destillationskolonne durchgeführt. Zum Reaktoreingang des ersten Reaktors wurde unter Reaktionsbedingungen ein Gemisch aus Raffinat II gemäß obiger Zusammensetzung geleitet. Zusätzlich wurde ein Wälzstrom (Reaktorausgangsstrom vom zweiten Reaktor) direkt wieder zum Reaktoreingang geleitet.

Der Katalysator wurde in beide Reaktoren eingefüllt und 24h unter Durchleiten von 30Nm³/h N₂ bei Normaldruck sowie einer Reaktortemperatur von 170°C getrocknet. Anschließend wurde der Katalysator unter folgenden Bedingungen betrieben: Raffinat II-feed (10kg/h), Wälzung (50kg/h), sowie Druck (30bar) und Temperatur (50°C). Unter diesen Bedingungen wurde ein C₄-Olefin-Umsatz von 55% erreicht, es resultierte ein C₈₊-Olefinaustrag, der aus 70% Butendimeren, 22% Butentrimeren, 7% ButenTetrameren und 1 % C20+-Olefinen bestand. Der C₈₊-Austrag wurde destilliert. Man erhielt ein Oligomer-Gemisch, das aus 7% Butentrimeren, 70% Butentetrameren, 17% Butenpentameren, 5% Butenehexameren und 1 % Buteneheptameren bestand (nachstehend "Buten-Oligomer-Mischung" genannt).

### Beispiele 1 bis 6

### Herstellung von Addukten aus Buten-Oligomer-Mischung und Maleinsäureanhydrid

In einem 1,2-I-Autoklaven wurden jeweils die in Tabelle 1 angegebenen Mengen an Buten-Oligomer-Mischung und 98g (1 mol) Maleinsäureanhydrid (MSA) unter einer Stickstoffatmosphäre vorgelegt, nach dem Verschließen des Autoklav auf eine Temperatur von 220°C mit einem Metallbad erhitzt und bei dieser Temperatur 5 Stunden gerührt. Der Druck stiegt bis auf 1,2 - 1,3 bar. Nach 5 Stunden wurde der Autoklav abgekühlt. Man erhielt eine braune, niederviskose Flüssigkeit. Die niedrigsiedenden Anteile wurden am Rotationsverdampfer bei 10 mbar und einer Innentemperatur bis 180°C abgezogen. Es wurde eine braune, geruchlose, viskose Flüssigkeit erhalten. Die Molverhältnisse und Auswaagen sind in Tabelle 1 aufgeführt.

**Tabelle 1**

| Beispiel | Menge Buten-Oligomer-Mischung | Molverhältnis von Buten-Oligomer-Mischung : MSA | Isolierte Menge |
|---|---|---|---|
| 1 | 233 g | ∼1 : 1 | 249 g (75%) |
| 2 | 256 g | ~1,1 : 1 | 258 g (73%) |
| 3 | 303 g | ~1,3 : 1 | 278 g (69%) |
| 4 | 373 g | ∼1,6 : 1 | 295 g (63%) |
| 5 | 419 g | ∼1,8 : 1 | 318 g (61%) |
| 6 | 466 g | ∼2 : 1 | 328 g (58%) |

### Beispiele 7 bis 9

### Herstellung von Addukten aus Buten-Oligomer-Mischung und Maleinsäureanhydrid bei verschiedenen Temperaturen

In einem 1,2-I-Autoklaven wurden 224g einer Buten-Oligomer-Mischung und 98g (1 mol) Maleinsäureanhydrid (MSA) unter Stickstoff vorgelegt, nach dem Verschließen des Autoklav auf die in Tabelle 2 jeweils angegebene Temperatur in einem Metallbad erhitzt und bei dieser Temperatur 5 Stunden gerührt. Dann wurde der Autoklav abgekühlt. Man erhielt eine braune niederviskose Flüssigkeit. Die niedrigsiedenden Anteile wurden am Rotationsverdampfer bei 1 mbar und einer Innentemperatur bis 160°C abgezogen. Es wurde eine braune, geruchlose, viskose Flüssigkeit erhalten. Die Auswaagen sind in Tabelle 2 aufgeführt.

**Tabelle 2**

| Beispiel | Druck / bar | Temperatur / °C | Isolierte Menge |
|---|---|---|---|
| 7 | 1,0 | 200 | 182 g (56%) |
| 8 | 1,3 | 220 | 243 g (75%) |
| 9 | 6,0 | 250°C | 255 g (79%) |

### Beispiele 10 bis 14

### Herstellung von Addukten aus Buten-Oligomer-Mischung und Maleinsäureanhydrid bei verschiedenen Reaktionszeiten

In einem 1,2-I-Autoklaven wurden 233 g Buten-Oligomer-Mischung und 98g (1 mol) Maleinsäureanhydrid (MSA) unter einer Stickstoffatmosphäre vorgelegt, nach dem Verschließen des Autoklav auf eine Temperatur von 220°C mit einem Metallbad erhitzt und bei dieser Temperatur gerührt. Nach der in Tabelle 3 jeweils angegebenen Zeit wurde die Reaktion abgebrochen, indem man den Autoklav abkühlte und das Reaktionsgemisch isolierte. Man erhielt eine braune leichtviskose Flüssigkeit. Die niedrigsiedenden Anteile wurden durch Erhitzen des Reaktionsgemisches am Rotationsverdampfer bei 10 mbar bis zu einer Temperatur von 180°C abgezogen. Man erhielt jeweils eine braune, geruchlose, viskose Flüssigkeit. Die Auswaagen (isolierte Mengen) sind in Tabelle 3 aufgeführt.

**Tabelle 3**

| Beispiel | Druck / bar | Zeit / Stunde | Isolierte Menge |
|---|---|---|---|
| 10 | 1,3 | 1 | 194 g (59%) |
| 11 | 1,3 | 4 | 240 g (73%) |
| 12 | 1,2 | 5 | 249 g (75%) |
| 13 | 1,3 | 6 | 257 g (78%) |
| 14 | 1,7 | 8 | 273 g (82%) |

### Beispiel 15

### Destillation eines Addukts von MSA an eine Buten-Oligomer-Mischung

240 g des nach Beispiel 8 hergestellten Adduktes von MSA an Buten-Oligomer-Mischung wurde in einem 500ml Kolben über eine 40cm Kolonne bei einem Druck von 1 mbar destilliert. Als Wärmeübertragungsmittel diente ein Metallbad. 3 Fraktionen und ein Vorlauf wurde bis 230°C Übergangtemperatur gesammelt. Der Sumpf war eine schwarze, hochviskose Flüssigkeit. Der Vorlauf und die drei Fraktionen waren gelbe, leichtviskose Flüssigkeiten. Destillationsdaten sind in Tabelle 4 aufgeführt.

**Tabelle 4**

| Fraktionen | Badtemperatur / °C | Übergangtemperatur / °C | Isolierte Menge / g |
|---|---|---|---|
| Vorlauf | 180 | 142 | 5 g (2%) |
| Fraktion 1 | 200 | 160 | 81 g (34%) |
| Fraktion 2 | 215 | 190 | 68 g (28%) |
| Fraktion 3 | 270 | 230 | 21 g (9%) |
| Sumpf | - | | 65 g (27%) |

### Beispiel 16

### Destillation der Buten-Oligomer-Mischung

3l (2360g) der oben beschriebenen Buten-Oligomer-Mischung wurden in einem 41 Kolben über eine Brücke bei einem Druck von 10mbar destilliert. Als Wärmeübertragungsmittel diente ein Ölbad. Als Destillat sammelte man insgesamt 1711g einer Buten-Oligomer-Mischung bis zu einer Übergangstemperatur von180°C. Es bestand aus 10% Butentrimeren, 78% Butentetrameren, 10% Butenpentameren und 2% Butenhexameren.

### Beispiele 17

### Herstellung von Addukten aus Destillat von Buten-Oligomer-Mischung (erhalten nach Beispiel 16)

In einem 1,2-I-Autoklaven wurden 224 g Destillat von Buten-Oligomer-Mischung gemäß Beispiel 16 und 98g (1 mol) Maleinsäureanhydrid (MSA) unter einer Stickstoffatmosphäre vorgelegt, nach dem Verschließen des Autoklav auf eine Temperatur von 220°C mit einem Metallbad erhitzt und bei dieser Temperatur 5 Stunden gerührt. Nach 5 Stunden wurde die Reaktion abgebrochen, indem man den Autoklav abkühlte und das Reaktionsgemisch isolierte. Man erhielt eine braune leichtviskose Flüssigkeit. Die niedrigsiedenden Anteile wurden durch Erhitzen des Reaktionsgemisches am Rotationsverdampfer bei 10 mbar bis zu einer Temperatur von 180°C abgezogen. Man erhielt jeweils eine braune, geruchlose, viskose Flüssigkeit. Die Auswaage war 261g (81%).

### Beispiel 18

### Herstellung von Addukten aus Destillat von Buten-Oligomer-Mischung

In einem 1,2-I-Autoklaven wurden 336 g einer reinen C₁₆ Buten-Oligomer-Mischung (100% C₁₆ Olefinen) und 98 g (1 mol) Maleinsäureanhydrid (MSA) unter einer Stickstoffatmosphäre vorgelegt, nach dem Verschließen des Autoklav auf eine Temperatur von 210 °C mit einem Metallbad erhitzt und bei dieser Temperatur 5 Stunden gerührt. Nach 5 Stunden wurde die Reaktion abgebrochen, indem man den Autoklav abkühlte und das Reaktionsgemisch isolierte. Man erhielt eine braune leichtviskose Flüssigkeit. Die niedrigsiedenden Anteile wurden durch Erhitzen des Reaktionsgemisches am Rotationsverdampfer bei 1 mbar bis zu einer Temperatur von 180°C abgezogen. Man erhielt jeweils eine braune, geruchlose, viskose Flüssigkeit. Die Auswaage war 192,7g und das Produkt enthielt nur Alkenylsuccinicanhydrid.

Als Nebenprodukte wurden 17,8 g Rest-MSA und 223,5 g Rest-Olefin abgetrennt.

### Anwendungstechnische Beispiele

### Herstellung von ASA Emulsionen

Eine 5%ige Suspension einer kationisierten Stärke (Hicat® 5163A, Fa. Roquette) wurde 30 Minuten in einem Kolben mit mechnischem Rührer unter Rückfluß gekocht bis sich die Stärke rückstandsfrei aufgelöst hatte. Anschließend wurde die Stärkelösung im Eisbad auf Raumtemperatur abgekühlt und mit Ameisensäure (1 % in Wasser) auf pH 4 eingestellt.

Zur Herstellung der ASA Emulsion wurden 200 g der Stärkelösung in einen Standmixer mit Glaskrug (Fa. ABC Elektro, Modell 260) überführt und jeweils 2 g des in Tabelle 5 angegebenen MSA/Olefin Addukts (ASA) hinzugegeben. Die Emulgierung erfolgte 30 Sekunden bei voller Leistung und 90 Sekunden bei halber Leistung. Die Messung der Teilchengrößenverteilung erfolgte auf einem Gerät der Fa. Coulter, Modell LS130. In Tabelle 5 sind die Ergebnisse der Emulgierversuche zusammengefasst:

**Tabelle 5**

| Beispiel | MSA/OlefinAddukt (ASA) hergestellt gemäß | D50 [µm] |
|---|---|---|
| 19 | Beispiel 1 | 1,6 |
| 20 | Beispiel 3 | 1,4 |
| 21 | Beispiel 4 | 1,2 |
| 22 | Beispiel 6 | 1,1 |
| 23 | Beispiel 18 | 1,5 |

### Anwendung der Emulsionen zur Leimung von Papier und Karton

In einer ersten Versuchsreihe wurde eine Zellstoffsuspension, bestehend aus Birken- und Kiefernsulfat, hergestellt. Diese wurde mit 20% gemahlenem Calciumcarbonat und 0,6% einer kationischen Massestärke versetzt. Anschließend wurden die oben beschriebenen ASA-Emulsionen zugesetzt. Nach Zugabe eines Retentionsmittels auf Basis Polyacrylamid wurden jeweils Blätter mittels eines Rapid-Köthen-Blattbildners hergestellt. Die so hergestellten Blätter wurden an einem Trockenzylinder getrocknet. Die Messung wurde nach Klimatisieren bei 50% Feuchte über 24h ausgeführt.

**Tabelle 6**

| ASA- Emulsion gemäß | Dosiermenge ASA [kg/t] | Cobb 60" [g/m²] | Tintenschwimmdauer [Min] |
|---|---|---|---|
| Beispiel 19 | 2 | 32 | 10 |
| Beispiel 20 | 2 | 27 | 11 |
| Beispiel 21 | 2 | 25 | 15 |
| Beispiel 22 | 2 | 24 | 19 |
| Beispiel 23 | 2 | 29 | 25 |

In einer weiteren Versuchsreihe wurde eine Stoffsuspension hergestellt, die aus 100% Altpapier bestand. Diese wurde zunächst mit 0,8% einer kationischen Massestärke versetzt. Anschließend wurden die oben beschriebenen ASA-Dispersionen, die gemäß den Beispielen 19 - 22 hergestellt wurden, zugesetzt. Nach Zugabe eines Retentionsmittels auf Basis Polyacrylamid wurden jeweils Blätter mittels eines Rapid-Köthen-Blattbildners hergestellt. Die so hergestellten Blätter wurden bei 90°C an einem Trockenzylinder getrocknet und dann bei 50% Feuchte über 24h klimatisiert. Anschließend wurde die Leimung nach Tintenschwimmdauer und Cobb 60 bestimmt. Die Ergebnisse sind in Tabelle 7 angegeben.

**Tabelle 7**

| ASA Emulsion gemäß | Dosiermenge ASA [kg/t] | Cobb 60" [g/m²] | Tintenschwimmdauer [Min] |
|---|---|---|---|
| Beispiel 19 | 3 | 41 | 23 |
| Beispiel 20 | 3 | 35 | 27 |
| Beispiel 21 | 3 | 35 | 33 |
| Beispiel 22 | 3 | 34 | 38 |

In einer weiteren Versuchsreihe wurde eine Zellstoffsuspension, bestehend aus gebleichtem Birkensulfat und Kiefernsulfat, hergestellt. Diese wurde zunächst mit 0,75% einer kationischen Massestärke versetzt. Anschließend wurden die in den Beispielen 19 - 22 beschriebenen ASA-Emulsionen zugesetzt. Nach Zugabe eines Retentionsmittels auf Basis Polyacrylamid wurden jeweils Blätter mittels eines Rapid-Köthen-Blattbildners mit einem Flächengewicht von 150g/m² hergestellt. Die so hergestellten Blätter wurden bei 90°C an einem Trockenzylinder getrocknet und dann bei 50% Feuchte über 24h klimatisiert. Anschließend wurden die Blätter von beiden Seiten mit einem Klebeband streifenfrei beschichtet. Aus den Blättern wurden Streifen einer Länge von 25 x 75mm geschnitten. Die Teststreifen wurden in ein Wasserstoffperoxid-Bad bei 70°C getaucht, um die Kantenpenetration durch Differenzwägung zu ermitteln. Die Ergebnisse sind in Tabelle 8 angegeben.

**Tabelle 8**

| ASA-Emulsion gemäß | Dosiermenge ASA [kg/t] | Kantenpenetration Peroxid, 70°C [kg/m²] |
|---|---|---|
| Beispiel19 | 3 | 3,25 |
| Beispiel 20 | 3 | 3,30 |
| Beispiel 21 | 3 | 2,70 |
| Beispiel 22 | 3 | 2,25 |

## Patentansprüche

1. Alkenylbernsteinsäureanhydride, die durch Umsetzung von Oligomeren von C₄-bis C₈-Olefinen und Maleinsäureanhydrid herstellbar sind, **dadurch gekennzeichnet, dass** man bei der Umsetzung mit Maleinsäureanhydrid Mischungen von Oligomeren mit mindestens 12 C-Atomen einsetzt, die erhältlich sind durch Oligomerisierung eines Kohlenwasserstoffgemisches, das mindestens zwei Olefine mit 4 bis 8 C-Atomen enthält, an einem Katalysator, der ein Übergangsmetall enthält, und wobei man bei der Oligomerisierung ein Kohlenwasserstoffgemisch einsetzt, das 20 bis 100 Gew.-% C₄-Olefine, 0 bis 80 Gew.-% C₅-Olefine, 0 bis 60 Gew.-% C₆-Olefine und 0 bis 10 Gew.-% von den zuvor genannten Olefinen verschiedene Olefine, jeweils bezogen auf den Gesamtolefingehalt, enthält.

2. Alkenylbernsteinsäureanhydride nach Anspruch 1, **dadurch gekennzeichnet, dass** man bei der Oligomerisierung ein Kohlenwasserstoffgemisch einsetzt, das einen Gehalt an linearen Monoolefinen von mindestens 80 Gew.-%, bezogen auf den Gesamtolefingehalt, aufweist.

3. Alkenylbernsteinsäureanhydride nach einem der Ansprüche 1 oder 2, **dadurch gekennzeichnet, dass** man bei der Oligomerisierung ein C₄-Olefingemisch einsetzt.

4. Alkenylbernsteinsäureanhydride nach einem der Ansprüche 1 bis 3, **dadurch gekennzeichnet, dass** man bei der Oligomerisierung ein C₄-Olefingemisch einsetzt, das 1-Buten und 2-Buten in einem Gewichtsverhältnis von 20 : 1 bis 1 : 2 enthält und aus dem Oligomerengemisch Trimere, Tetramere, Pentamere und/oder Hexamere isoliert.

5. Alkenylbernsteinsäureanhydride nach einem der Ansprüche 1 bis 4, **dadurch gekennzeichnet, dass** man bei der Oligomerisierung ein Kohlenwasserstoffgemisch einsetzt, das
| | |
|---|---|
| 0,5 bis 5 Gew.-% | Isobutan, |
| 5 bis 20 Gew.-% | n-Butan, |
| 20 bis 40 Gew.-% | trans-2-Buten, |
| 10 bis 20 Gew.-% | cis-2-Buten, |
| 25 bis 55 Gew.-% | 1-Buten und |
| 0,5 bis 5 Gew.-% | Isobuten |
enthält.

6. Verfahren zur Herstellung von Alkenylbernsteinsäureanhydriden durch Umsetzung von Oligomeren von C₄- bis C₈-Olefinen und Maleinsäureanhydrid, **dadurch gekennzeichnet, dass** man bei der Umsetzung mit Maleinsäureanhydrid Mischungen von Oligomeren mit mindestens 12 C-Atomen einsetzt, die erhältlich sind durch Oligomerisierung eines Kohlenwasserstoffgemisches, das mindestens zwei Olefine mit 4 bis 8 C-Atomen enthält, an einem Katalysator, der ein Übergangsmetall enthält, und wobei man bei der Oligomerisierung ein Kohlenwasserstoffgemisch einsetzt, das 20 bis 100 Gew.-% C₄-Olefine, 0 bis 80 Gew.-% C₅-Olefine, 0 bis 60 Gew.-% C₆-Olefine und 0 bis 10 Gew.-% von den zuvor genannten Olefinen verschiedene Olefine, jeweils bezogen auf den Gesamtolefingehalt, enthält.

7. Verwendung der Mischungen von Alkenylbernsteinsäureanhydriden nach einem der Ansprüche 1 bis 5 als Leimungsmittel für Papier.

8. Verwendung nach Anspruch 7, **dadurch gekennzeichnet, dass** man eine Mischung von Alkenylbernsteinsäureanhydriden einsetzt, die erhältlich ist durch
(i) Oligomerisierung eines Kohlenwässerstoffgemisches, das
| | |
|---|---|
| 0,5 bis 5 Gew.-% | Isobutan, |
| 5 bis 20 Gew.-% | n-Butan, |
| 20 bis 40 Gew.-% | trans-2-Buten. |
| 10 bis 20 Gew.-% | cis-2-Buten, |
| 25 bis 55 Gew.-% | 1-Buten und |
| 0,5 bis 5 Gew.-% | Isobuten |
enthält zu einem n-Butan und Isobutan enthaltendem Gemisch von Buten-Oligomeren und
(ii) Umsetzung dieses Gemisches mit Maleinsäureanhydrid zu einer Mischung von C₁₆- bis C₂₄-Alkenylbernsteinsäureanhydriden.

## Claims

1. An alkenylsuccinic anhydride which can be prepared by reacting oligomers of C₄- C₈-olefins and maleic anhydride, wherein mixtures of oligomers having at least 12 carbon atoms which are obtainable by oligomerization of a hydrocarbon mixture which comprises at least two olefins having 4 to 8 carbon atoms over a catalyst which comprises a transition metal are used in the reaction with maleic anhydride, and a hydrocarbon mixture which comprises from 20 to 100% by weight of C₄-olefins, from 0 to 80% by weight of C₅-olefins, from 0 to 60% by weight of C₆-olefins and from 0 to 10% by weight of olefins differing from the abovementioned olefins, based in each case on the total olefin content, being used in the oligomerization.

2. The alkenylsuccinic anhydride according to claim 1, wherein a hydrocarbon mixture which has a content of at least 80% by weight, based on the total olefin content, of linear monoolefins is used in the oligomerization.

3. The alkenylsuccinic anhydride according to either of claims 1 or 2, wherein a C₄-olefin mixture is used in the oligomerization.

4. The alkenylsuccinic anhydride according to any of claims 1 to 3, wherein a C₄-olefin mixture which comprises 1-butene and 2-butene in a weight ratio of from 20:1 to 1:2 is used in the oligomerization and trimers, tetramers, pentamers and/or hexamers are isolated from the oligomer mixture.

5. The alkenylsuccinic anhydride according to any of claims 1 to 4, wherein a hydrocarbon mixture which comprises
from 0.5 to 5% by weight of isobutane,
from 5 to 20% by weight of n-butane,
from 20 to 40% by weight of trans-2-butene,
from 10 to 20% by weight of cis-2-butene,
from 25 to 55% by weight of 1-butene and
from 0.5 to 5% by weight of isobutene
is used in the oligomerization.

6. A process for the preparation of alkenylsuccinic anhydrides by reacting oligomers of C₄- to C₈-olefins and maleic anhydride, wherein mixtures of oligomers having at least 12 carbon atoms which are obtainable by oligomerization of a hydrocarbon mixture which comprises at least two olefins having 4 to 8 carbon atoms over a catalyst which comprises a transition metal are used in the reaction with maleic anhydride, and a hydrocarbon mixture which comprises from 20 to 100% by weight of C₄-olefins, from 0 to 80% by weight of C₅-olefins, from 0 to 60% by weight of C₆-olefins and from 0 to 10% by weight of olefins differing from the abovementioned olefins, based in each case on the total olefin content, being used in the oligomerization.

7. The use of mixtures of alkenylsuccinic anhydrides according to any of claims 1 to 5 as sizes for paper.

8. The use according to claim 7, wherein a mixture of alkenylsuccinic anhydrides is used which is obtainable by
(i) oligomerization of a hydrocarbon mixture which comprises
from 0.5 to 5% by weight of isobutane,
from 5 to 20% by weight of n-butane,
from 20 to 40% by weight of trans-2-butene,
from 10 to 20% by weight of cis-2-butene,
from 25 to 55% by weight of 1-butene and
from 0.5 to 5% by weight of isobutene
to give a mixture of butene oligomers which comprises n-butane and isobutane and
(ii) reaction of this mixture with maleic anhydride to give a mixture of C₁₆- to C₂₄-alkenylsuccinic anhydrides.

## Revendications

1. Anhydrides d'acides alcényle succiniques, qui peuvent être fabriqués par réaction d'oligomères d'oléfines en C₄-C₈ et d'anhydride de l'acide maléique, **caractérisés en ce que** lors de la réaction avec l'anhydride de l'acide maléique, des mélanges d'oligomères comportant au moins 12 atomes C sont utilisés, qui peuvent être obtenus par oligomérisation d'un mélange d'hydrocarbures qui contient au moins deux oléfines comportant 4 à 8 atomes C, sur un catalyseur qui contient un métal de transition, et **en ce que** lors de l'oligomérisation, un mélange d'hydrocarbures, qui contient 20 à 100 % en poids d'oléfines en C₄, 0 à 80 % en poids d'oléfines en C₅, 0 à 60 % en poids d'oléfines en C₆ et 0 à 10 % en poids d'oléfines différentes des oléfines susmentionnées, à chaque fois par rapport à la teneur totale en oléfines, est utilisé.

2. Anhydrides d'acides alcényle succiniques selon la revendication 1, **caractérisés en ce qu'**un mélange d'hydrocarbures qui présente une teneur en monooléfines linéaires d'au moins 80 % en poids, par rapport à la teneur totale en oléfines, est utilisé lors de l'oligomérisation.

3. Anhydrides d'acides alcényle succiniques selon l'une quelconque des revendications 1 ou 2, **caractérisés en ce qu'**un mélange d'oléfines en C₄ est utilisé lors de l'oligomérisation.

4. Anhydrides d'acides alcényle succiniques selon l'une quelconque des revendications 1 à 3, **caractérisés en ce qu'**un mélange d'oléfines en C₄ qui contient du 1-butène et du 2-butène en un rapport en poids de 20:1 à 1:2 est utilisé lors de l'oligomérisation et des trimères, des tétramères, des pentamères et/ou des hexamères sont isolés du mélange d'oligomères.

5. Anhydrides d'acides alcényle succiniques selon l'une quelconque des revendications 1 à 4, **caractérisés en ce qu'**un mélange d'hydrocarbures qui contient
0,5 à 5 % en poids d'isobutane,
5 à 20 % en poids de n-butane,
20 à 40 % en poids de trans-2-butène,
10 à 20 % en poids de cis-2-butène,
25 à 55 % en poids de 1-butène et
0,5 à 5 % en poids d'isobutène
est utilisé lors de l'oligomérisation.

6. Procédé de fabrication d'anhydrides d'acides alcényle succiniques par réaction d'oligomères d'oléfines en C₄-C₈ et d'anhydride de l'acide maléique, **caractérisé en ce que** lors de la réaction avec l'anhydride de l'acide maléique, des mélanges d'oligomères comportant au moins 12 atomes C sont utilisés, qui peuvent être obtenus par oligomérisation d'un mélange d'hydrocarbures qui contient au moins deux oléfines comportant 4 à 8 atomes C, sur un catalyseur qui contient un métal de transition, et **en ce que** lors de l'oligomérisation, un mélange d'hydrocarbures, qui contient 20 à 100 % en poids d'oléfines en C₄, 0 à 80 % en poids d'oléfines en C₅, 0 à 60 % en poids d'oléfines en C₆ et 0 à 10 % en poids d'oléfines différentes des oléfines susmentionnées, à chaque fois par rapport à la teneur totale en oléfines, est utilisé.

7. Utilisation des mélanges d'anhydrides d'acides alcényle succiniques selon l'une quelconque des revendications 1 à 5 en tant qu'agent de collage pour le papier.

8. Utilisation selon la revendication 7, **caractérisée en ce qu'**un mélange d'anhydrides d'acides alcényle succiniques est utilisé, qui peut être obtenu par
(i) oligomérisation d'un mélange d'hydrocarbures qui contient
0,5 à 5 % en poids d'isobutane,
5 à 20 % en poids de n-butane,
20 à 40 % en poids de trans-2-butène,
10 à 20 % en poids de cis-2-butène,
25 à 55 % en poids de 1-butène et
0,5 à 5 % en poids d'isobutène
pour former un mélange d'oligomères de butène contenant du n-butane et de l'isobutane et
(ii) réaction de ce mélange avec de l'anhydride de l'acide maléique pour former un mélange d'anhydrides d'acides alcényle succiniques en C₁₀ à C₂₄.
